# FASCICULE DE BREVET EUROPEEN

(11) **EP 3 657 979 B1**
(45) Date de publication et mention de la délivrance du brevet: **31.12.2025**
(21) Numéro de dépôt: 18752599.3
(22) Date de dépôt: 20.07.2018
(51) Int. Cl.: A45D 40/04

(54) **DISPOSITIF DE DISTRIBUTION D'UN PRODUIT COSMÉTIQUE OU DE SOIN COMPORTANT DES ÉLÉMENTS SOLIDES ROMPUS LORS DE LA DISTRIBUTION**
VORRICHTUNG ZUR AUSGABE EINES KOSMETISCHEN ODER PFLEGEPRODUKTS MIT WÄHREND DER AUSGABE ZERBRECHENDEN FESTSTOFFELEMENTEN
DEVICE FOR DISPENSING A COSMETIC OR CARE PRODUCT COMPRISING SOLID ELEMENTS THAT ARE BROKEN DURING DISPENSING

(30) Priorité: 26.07.2017 FR 1757087
(43) Date de publication de la demande: 03.06.2020
(73) Titulaire: Chanel Parfums Beauté, 92200 Neuilly-sur-Seine (FR)
(72) Inventeur: CASTEX, Nicolas, 92700 Colombes (FR); PERONNE, Floriane, 75017 Paris (FR); CAYRON, Antoine, 1190 Forest (BE)
(74) Mandataire: Santarelli
(86) Numéro de dépôt international: PCT/FR2018/051864
(87) Numéro de publication internationale: WO 2019/020913

(56) Documents cités:
- EP-B1- 3 657 978
- WO-A1-2012/145003
- WO-A1-2017/108706
- DE-A1- 102015 106 414
- FR-A1- 2 994 536
- JP-A- 2002 192 005
- JP-A- 2014 046 937
- US-A1- 2009 283 545

## Description

L'invention concerne le domaine des dispositifs de distribution et d'application d'un produit cosmétique ou de soin.

Les produits cosmétiques englobent tous les produits pour le maquillage de la peau ou des phanères ainsi que toutes les compositions parfumantes ou odorantes destinées à une application corporelle.

Les produits de soin comportent notamment les produits destinés à être appliqués sur le corps humain ou animal pour traiter ou prévenir une pathologie.

L'invention porte en particulier sur un dispositif de distribution et d'application d'un tel produit cosmétique ou de soin se présentant sous forme liquide, semi-fluide ou pâteuse.

L'invention présente un intérêt particulier pour l'application d'un mélange hétérogène contenant des éléments solides dispersés, en suspension. Un tel mélange peut par exemple comporter des particules solides ou des billes pouvant comporter un coeur liquide entouré d'une membrane gélifiée. Par exemple, certaines compositions cosmétiques ou de soin comportent des principes actifs, essences ou autres ingrédients ainsi encapsulés.

Les particules ou billes d'un tel mélange hétérogène doivent cependant être rompues afin de libérer leur contenu lors de la distribution du mélange, c'est-à-dire soit lors de l'application du mélange sur l'utilisateur, ou juste avant cette application par exemple lors de la migration du mélange entre un réservoir et un applicateur.

Le cas échéant, le contenu des particules ou billes doit être homogénéisé avec le reste du mélange.

De tels fluides peuvent être délivrés, de manière connue, au moyen de pompes présentant une capacité d'aspiration élevée ou à l'aide d'une pompe sans air.

Ces pompes présentent cependant l'inconvénient d'être relativement coûteuses, et peuvent se révéler inadaptées à l'aspiration de solution contenant des solides en suspension. Aussi, les solides en suspension peuvent bloquer la pompe et l'endommager, rendant le dispositif de distribution inopérant.

Il a été envisagé dans l'état de la technique de doter les dispositifs de distribution d'éléments du type grilles ou filtres, destinés à briser, broyer et/ou mélanger les éléments solides en suspension dans une composition liquide ou pâteuse hétérogène. Le document US5284275 présente un élément de délivrance d'une composition adhésive formée par le mélange de billes d'un premier produit dans un second produit. L'élément décrit comporte une paroi de filtration perpendiculaire à la direction de circulation du produit dans l'élément de délivrance. Un piston permet de pousser les billes au travers de la paroi de filtration, ce qui les rompt. Un tel dispositif est cependant facilement sujet au colmatage de la paroi de filtration.

Un moyen de distribution, résolvant en partie ce problème, est par ailleurs divulgué dans le document FR2994536. Le dispositif divulgué dans ce document comporte un conduit de guidage de la composition à délivrer, doté d'un organe de filtration comportant un organe de filtration à paroi de filtration typiquement conique, formant ainsi un angle non-nul avec l'axe du conduit de guidage.

L'organe de filtration, sous la pression d'un piston, permet de provoquer la rupture des membranes des billes qui viennent interférer avec la paroi de filtration au niveau des ouvertures qu'elle comporte.

Néanmoins, si un tel organe de filtration permet de délivrer une quantité importante de produit, il ne permet pas de garantir que toutes les billes (ou autres éléments solides) ni même qu'une majorité des billes contenues dans la composition sont rompues au passage de l'organe de filtration.

Un dispositif de distribution comportant un tel organe de filtration est ainsi peu approprié à la distribution de certains produits pour lesquels il faut s'assurer qu'un certain pourcentage, majoritaire, des billes est éclaté avant l'application du produit. C'est par exemple le cas pour un rouge à lèvres ou un brillant à lèvres (généralement désigné par le terme anglophone « gloss »), car l'application finale par l'utilisateur sur ses lèvres ne permet pas de rompre les billes qui ne l'ont pas été avant application.

L'invention vise à proposer un dispositif de distribution d'un produit cosmétique ou de soin liquide ou pâteux et initialement hétérogène, comportant des éléments solides en suspension, permettant de maximiser et de fiabiliser la quantité d'éléments solides rompus lors de la distribution du produit, et d'homogénéiser le produit, tout en limitant le risque de colmatage.

Ainsi, l'invention porte sur un dispositif de distribution d'un produit cosmétique ou de soin liquide, semi-fluide ou pâteux, stocké sous forme hétérogène comportant des éléments solides en suspension pouvant être rompus mécaniquement pour libérer un fluide qu'ils contiennent, comportant un réservoir destiné au stockage du produit, un embout de distribution du produit, un conduit de guidage permettant le transport du produit entre le réservoir et l'embout de distribution, et des moyens adaptés à provoquer la circulation du produit du réservoir vers l'embout, via le conduit de guidage, le conduit de guidage comportant un dispositif de rupture des éléments solides. Le dispositif de rupture d'élément solides comporte un premier étage, en sortie du réservoir, formant une première portion du conduit de guidage et comportant une section transversale qui évolue progressivement et qui présente en sortie des arêtes saillantes à l'intérieur dudit conduit de guidage, et un deuxième étage, à proximité de l'embout de distribution, formant une deuxième portion du conduit de guidage et présentant une restriction de section progressive, ladite deuxième portion du conduit de guidage, formée dans le deuxième étage (E2) du dispositif de rupture, est en forme de tronc de cône..

La demanderesse a constaté qu'un dispositif de rupture d'éléments solides comportant deux étages, dont chaque étage provoque la rupture desdits éléments selon des modalités différentes, permet l'obtention de la rupture de la majorité, voire de la quasi-totalité ou de la totalité des éléments solides traversant ce dispositif de rupture.

Les étages du dispositif de rupture formant des portions du conduit de guidage du produit entre le réservoir et l'embout de distribution du produit, le risque de colmatage du dispositif est limité, ainsi que la perte de charge induite par le dispositif de rupture des éléments solides, ce qui permet la délivrance aisée d'une quantité importante de produit.

Le dispositif peut en outre comporter, en aval du deuxième étage du dispositif de rupture, un clapet anti-retour. Le clapet anti-retour peut présenter une embouchure de sortie du produit de section inférieure à la section de sortie du premier étage et à la section la plus restreinte du deuxième étage.

La section de sortie du premier étage est par exemple en étoile.

La restriction de section du deuxième étage peut par exemple correspondre à une restriction comprise entre 50% et 95% de la surface de la section d'entrée du deuxième étage.

Le dispositif peut comporter un corps tournant allongé et creux et un fût extérieur allongé et creux aboutés l'un à l'autre, et mobiles en rotation l'un par rapport à l'autre, l'embout de distribution étant fixé en bout du corps tournant, lesdits corps tournant et fût extérieur formant un volume intérieur dans lequel sont ménagés : un cylindre percé, comportant le conduit de guidage, et un fût intérieur, comportant une extrémité ouverte par laquelle est introduit le cylindre percé et une extrémité fermée de sorte à former le réservoir. Ce dispositif peut comporter un mécanisme de transformation d'une rotation du corps tournant par rapport au fût extérieur en un mouvement de translation du fût intérieur vis-à-vis du cylindre percé dans le volume intérieur formé par lesdits corps tournant et fût extérieur. Le dispositif peut en outre comporter un piston monté fixement à une extrémité du cylindre percé introduite par l'extrémité ouverte du fût intérieur et une portion allongée formant une portion du conduit de guidage dans le cylindre percé, dans lequel le piston comporte une ouverture centrale formant le premier étage du dispositif de rupture.

L'invention porte également sur un ensemble comportant un dispositif tel que décrit précédemment, et un produit cosmétique ou de soin comportant des éléments solides en suspension, pouvant être rompus mécaniquement pour libérer un fluide qu'ils contiennent, ledit produit cosmétique ou de soin étant stocké dans le réservoir du dispositif.

Dans un tel ensemble, les éléments solides peuvent être des billes sensiblement sphériques de diamètre inférieur à la dimension maximale de la section la plus restreinte du deuxième étage.

D'autres particularités et avantages de l'invention apparaîtront encore dans la description ci-après.

Aux dessins annexés, donnés à titre d'exemples non limitatifs :
- la figure 1 représente, selon une vue schématique en coupe, un dispositif de distribution d'un produit conforme à un mode de réalisation de l'invention ;
- la figure 2 représente, selon une vue éclatée, le dispositif de la figure 1 ;
- la figure 3 représente, selon une vue schématique coupée, en trois dimensions, certains éléments constitutifs du dispositif des figures 1 et 2 ;
- la figure 4 représente, selon une vue schématique en trois dimensions, un exemple de premier étage d'un dispositif de rupture d'éléments solides ;
- la figure 5 représente, selon une vue schématique tronquée, en trois dimensions, un exemple de deuxième étage d'un dispositif de rupture d'éléments solides ;
- la figure 6 représente, selon une vue schématique tronquée, en trois dimensions, un exemple de clapet anti-retour pouvant être mis en œuvre dans certains modes de réalisation de l'invention.

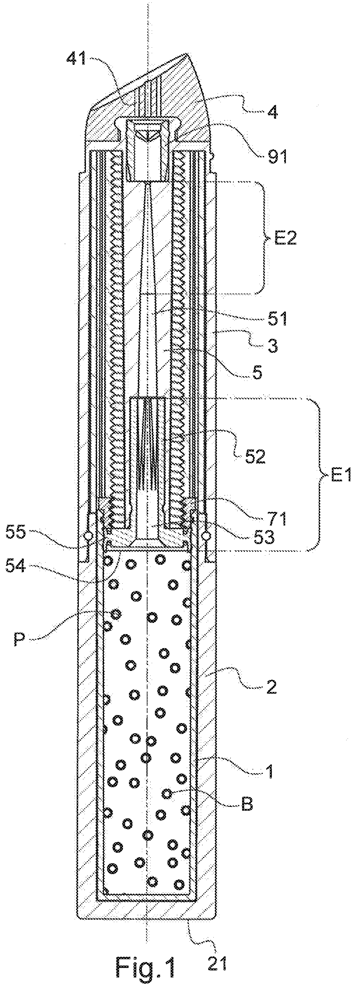

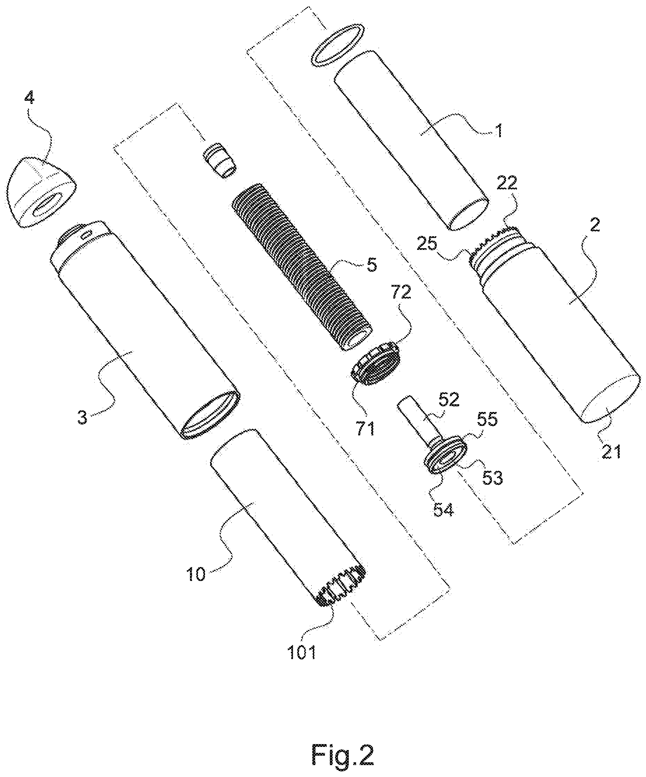

La figure 1 représente un dispositif conforme à un mode de réalisation de l'invention. La figure 2 représente en outre une vue éclatée du dispositif de la figure 1, qui en détaille les pièces constitutives et permet d'en appréhender l'assemblage.

Le dispositif permet la distribution et l'application d'un produit P cosmétique ou de soin, fluide, semi-fluide, ou pâteux. Le produit P est dit hétérogène, en ce qu'il comporte des éléments solides B en suspension. Les éléments solides B peuvent être rompus mécaniquement pour libérer un fluide qu'ils contiennent. Les éléments solides B peuvent par exemple être des billes comportant une coque gélifiée dans laquelle est contenu un ou plusieurs liquides, principes actifs, essences, huiles essentielles, ou autres ingrédients.

Les éléments solides B sont en suspension dans le produit lorsqu'il est stocké dans un réservoir 1 du dispositif, ou peuvent être mis en suspension par agitation du dispositif avant utilisation.

Dans l'exemple de mode de réalisation ici représenté, le réservoir 1 est formé par un élément appelé fût intérieur.

Le fût intérieur a la forme d'un cylindre droit fermé à une extrémité et ouvert à l'autre.

Le dispositif comporte un fût extérieur 2. Le fût extérieur 2 forme la partie inférieure du dispositif de distribution. Il est de forme allongée, et peut avoir une forme extérieure cylindrique ou prismatique. Le fût extérieur 2 comporte un fond 21 pouvant comporter une paroi de fermeture. Cette prise d'air peut être ménagée au travers de la paroi du fond 21. La prise d'air permet les échanges d'air entre l'intérieur et l'extérieur du fût extérieur 2. Elle permet notamment l'entrée d'air dans le fût extérieur lors du mouvement, ci-après décrit, du fût intérieur 1 dans le fût extérieur 2 pendant la distribution de produit. Le fût extérieur 2 comporte également une face opposée 22 ouverte, dans laquelle est introduit le fût intérieur. La forme intérieure du fût extérieur 2 correspond à un jeu près à la forme externe du fût intérieur. Cela permet une translation longitudinale, ou autrement dit un coulissement selon un axe principal (A) du dispositif, du fût intérieur faisant réservoir 1 dans le fût extérieur 2.

Le fût extérieur 2 est avantageusement transparent ou translucide, ce qui permet de visualiser la position du fût intérieur dans le dispositif. La position du fût intérieur dans le dispositif correspond en outre à une quantité donnée de produit restant. En d'autres termes, visualiser la position du fût intérieur permet à l'utilisateur de visualiser la quantité restante de produit, ou, réciproquement, la quantité consommée de produit.

Si le fût intérieur est lui-même transparent ou translucide, le produit est directement visible. Cela est esthétiquement valorisant, et permet en outre à l'utilisateur d'identifier le produit souhaité par son apparence, notamment sa couleur, sa brillance, et/ou ses autres caractéristiques visuelles, comme la présence des éléments solides en suspension pouvant avoir un aspect esthétique valorisant, par leur couleur, leur brillance, leur forme, etc.

Le dispositif comporte également un élément qui forme sa partie supérieure et qui est appelé corps tournant 3. Le corps tournant 3 est de forme allongée, et peut avoir une forme extérieure cylindrique ou prismatique, et notamment une forme extérieure correspondant à la forme extérieure du fût inférieur 2. Le corps tournant 3 est abouté au fût extérieur 2. Le corps tournant 3 peut être pivoté vis-à-vis de ce dernier autour de l'axe principal (A) du dispositif.

Le corps tournant 3 est désigné ainsi car il constitue l'élément que l'utilisateur tourne, par rapport au fût extérieur 2, afin de provoquer la distribution de produit cosmétique par le dispositif.

Le corps tournant 3 est creux et définit ainsi, avec le fût extérieur 2 auquel il est lié, un volume intérieur du dispositif de distribution.

Dans l'exemple ici représenté, un embout de distribution 4 est fixé en bout du corps tournant 3. L'embout de distribution 4 présente une surface d'application 41, par laquelle le produit cosmétique P est distribué et qui en permet l'application, par exemple en une couche mince et régulière sur la peau de l'utilisateur.

Le dispositif comporte, dans le volume intérieur formé par le corps tournant 3 et le fût extérieur 2, d'une part le fût intérieur, mais également un cylindre percé 5. Le cylindre percé 5 comporte sur toute sa longueur, c'est-à-dire le long de son axe d'extension confondu avec l'axe principal (A), un conduit longitudinal.

Dans l'exemple ici représenté, le cylindre percé 5 est fixe en translation par rapport au corps tournant 3, à l'intérieur duquel il s'étend. Le cylindre percé 5 est fixé au corps tournant 3, et fixe en rotation par rapport à ce dernier.

Le cylindre percé 5 est introduit dans le fût intérieur par son extrémité ouverte 22.

Le dispositif comporte un mécanisme de transformation d'une rotation du corps tournant 3 par rapport au fût extérieur 2 en un mouvement de translation du fût intérieur vis-à-vis du cylindre percé 5.

Ainsi, dans le volume intérieur formé par lesdits corps tournant et fût extérieur, c'est le fût intérieur qui est mobile, tandis que le cylindre percé 5 reste fixe longitudinalement. Du fait du mouvement du fût intérieur, le cylindre percé pénètre plus ou moins dans ledit fût intérieur, ce qui en fait varier le volume intérieur disponible dans le réservoir 1 pour le stockage du produit cosmétique P.

Une diminution du volume disponible dans le réservoir entraîne une expulsion d'une quantité correspondante de produit P via le conduit 51 du cylindre percé 5.

Le mécanisme de transformation du mouvement peut avoir diverses configurations. Ces mécanismes peuvent être basés, comme dans l'exemple ici représenté, sur une pièce filetée (présentant un filetage femelle ou mâle) liée au fût intérieur, et sur le fait que l'ensemble formé par le corps tournant 3 et le cylindre percé 5 comporte une partie filetée (mâle ou femelle, en correspondance) adaptée à coopérer avec la pièce filetée.

La rotation dudit ensemble formé par le corps tournant 3 et le cylindre percé 5 entraîne la translation de ladite pièce filetée et du fût intérieur 1 dans ledit corps tournant 3, et corollairement du fût intérieur 1 vis-à-vis du cylindre percé 5.

Dans l'exemple des figures 1 et 2 la pièce filetée est une bague 71 présentant un filetage intérieur, appelé filetage femelle. La bague 71 est fixée à l'extrémité ouverte 22 du fût intérieur, qu'elle obstrue si ce n'est au niveau de son ouverture filetée.

En remontant dans le corps tournant, le fût intérieur remonte le long du cylindre percé 5, ce qui a pour effet de diminuer le volume intérieur disponible dans le réservoir 1 pour le produit P, et de provoquer l'expulsion d'une quantité de produit P du fût intérieur par le conduit 51 du cylindre percé 5.

Le dispositif comporte un piston 52, qui est fixé à l'extrémité du cylindre percé. Le piston 52 comporte une ouverture centrale 53 qui communique avec le conduit 51 de sorte à former un conduit de guidage du produit P entre le réservoir 1 et l'embout de distribution 4.

L'ouverture centrale 53 assure ainsi une communication fluidique entre une surface frontale 54 du piston 52, qui est en contact avec le produit P dans le cylindre percé 5, et le reste du conduit 51. Le piston 52 est configuré de sorte à présenter une surface périphérique 55 en contact étanche avec la paroi intérieure du fût intérieur.

Afin de permettre la translation de la bague 71 le long de la partie filetée du cylindre creux 5, ladite bague 71 doit être libre en translation vis-à-vis du fût extérieur 2 et du corps tournant 3 tout en étant maintenue fixe en rotation vis-à-vis du fût extérieur 2. A cette fin, le dispositif comporte un manchon 10, s'étendant dans le corps tournant, qui est fixe par rapport au fût extérieur 2 et qui présente des guides en translation de la bague 71.

Ainsi, le fût extérieur 2 comporte en sa partie supérieure un crantage 25 adapté à s'engrener avec une denture correspondante 101 dudit manchon, ce qui bloque le manchon 10 en rotation vis-à-vis du fût extérieur 2. Le manchon 10 comporte sur sa surface interne des rainures longitudinales, c'est-à-dire parallèles à l'axe principal (A), le long desquelles la bague 71 est guidée. La bague 71 peut pour cela présenter, sur sa périphérie, des encoches 72 (figure 2) qui coulissent sur les rainures longitudinales.

Ainsi, lorsque le fût intérieur remonte autour du cylindre percé 5, la diminution de volume disponible dans le fût intérieur sous la surface frontale 54 du piston 52 entraîne l'expulsion d'une quantité de produit P correspondant à cette diminution.

Le produit est expulsé via l'ouverture centrale 53 du piston 52, puis via le conduit 51 du cylindre percé, avant d'atteindre l'embout d'application 4 qu'il traverse par des orifices distributeurs 42 avant d'atteindre la surface d'application 41.

Entre son stockage dans le réservoir 1 et sa distribution, le produit P traverse un dispositif de rupture d'éléments solides. Le dispositif de rupture d'éléments solides est destiné à rompre ou éclater les éléments solides contenant un fluide qui sont en suspension dans le produit P, qui est stocké sous forme hétérogène dans le réservoir 1, de sorte que le fluide se mêle au reste du produit P juste avant sa distribution. Une majorité d'éléments solides est rompue par le dispositif de rupture d'éléments solides, et c'est ainsi un produit relativement homogène qui est distribué (il peut cependant, selon certaines variantes de l'invention, rester des éléments solides non éclatés ou incomplètement rompus).

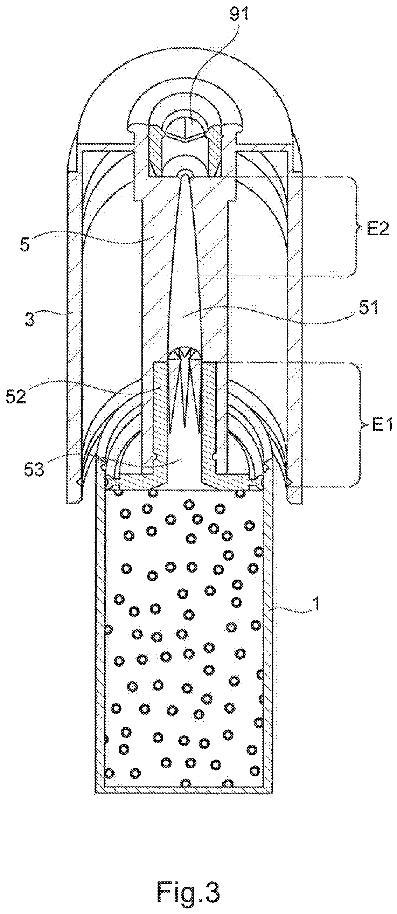

Le dispositif de rupture d'éléments solides comporte au moins deux étages, à savoir un premier étage E1 et un deuxième étage E2. Le dispositif de rupture d'éléments solides est formé dans le conduit de guidage qui comporte (ou est constitué par) l'ouverture centrale 53 du piston 52 et le conduit 51 du cylindre percé 5. Un exemple de dispositif de rupture, équipant le dispositif de distribution des figures 1 et 2, est en particulier représenté sur la vue en coupe tridimensionnelle de la figure 3.

On a représenté sur la figure 3 seulement certaines pièces constitutives d'un dispositif de distribution tel que représenté sur les figures 1 et 2. Sur la figure 3 sont représentés :
- le réservoir 1 ;
- le corps tournant 3 ;
- le cylindre percé 5 ;
- le piston 52 ; et
- un clapet anti-retour 91.

Le piston 52, et en particulier l'ouverture centrale 53 du piston 52 forme le premier étage E1 du dispositif de rupture d'éléments solides. Une portion extrême du conduit 51, proximale de l'embout de distribution 4, forme le deuxième étage E2 du dispositif de rupture d'éléments solides.

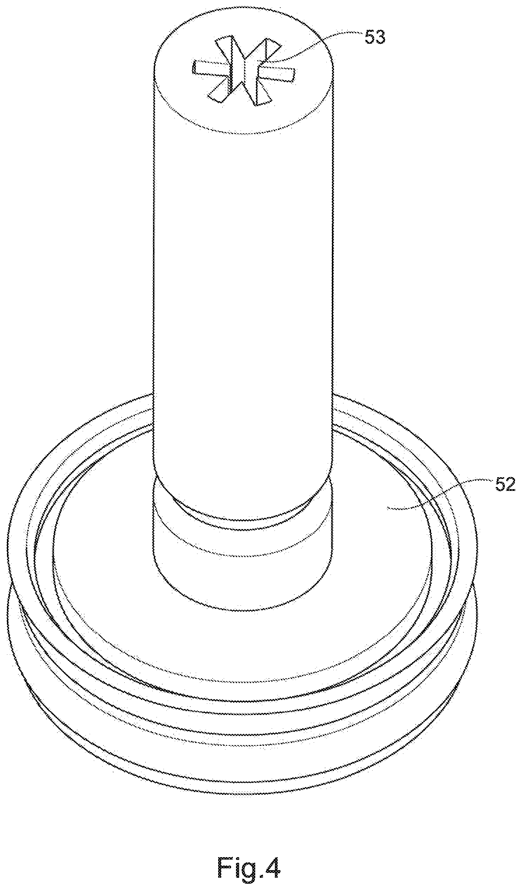

La figure 4, présentant une vue de détail du piston 52, permet avec la figure 3 de mieux comprendre la constitution du premier étage E1.

Le premier étage E1 du dispositif de rupture d'éléments solides est formé par l'ouverture centrale 53 du piston 52. L'ouverture centrale 53 est un conduit qui s'étend dans une portion allongée du piston 52 insérée dans le cylindre percé 5. Elle met en relation fluidique le réservoir 1 avec le conduit 51.

Dans l'exemple ici représenté, l'ouverture centrale présente au niveau du réservoir 1 une entrée ayant une section circulaire large, permettant le passage libre du produit P et notamment des éléments solides B qu'il comporte. Le diamètre de l'entrée de l'ouverture centrale 53 est ainsi largement supérieur à la dimension maximale des éléments solides B (par exemple le diamètre des éléments solides B s'il s'agit sensiblement de sphères).

En sortie, l'ouverture centrale 53 du piston 52 présente une forme en astérisque, tel que représenté à la figure 4. De nombreuses formes de sortie sont envisageables pour le premier étage E1 du dispositif de rupture d'éléments solides, lesdites formes comportant des arêtes saillantes à l'intérieur dudit conduit de guidage. Les formes possibles incluent celles d'étoiles, notamment d'étoiles à cinq, six, sept ou huit branches triangulaire. Les formes possibles incluent les croix et astérisques à cinq ou six branches.

L'évolution entre la forme d'entrée de l'ouverture centrale 53 et la forme de sa sortie est progressive, et s'étend sur une portion ou sur la totalité de la longueur de l'ouverture centrale 53.

La restriction de la section de sortie permet d'accélérer le flux de produit et de presser les éléments solides contre les arêtes saillantes dans l'ouverture centrale 53, ce qui tend à les rompre, notamment à rompre leur paroi extérieure.

Le deuxième étage du dispositif de rupture d'éléments solides est constitué d'une importante restriction de section du conduit de guidage. Dans l'exemple représenté, la restriction de section du conduit de guidage est localisée dans sa portion formée par le conduit 51 du cylindre percé 5.

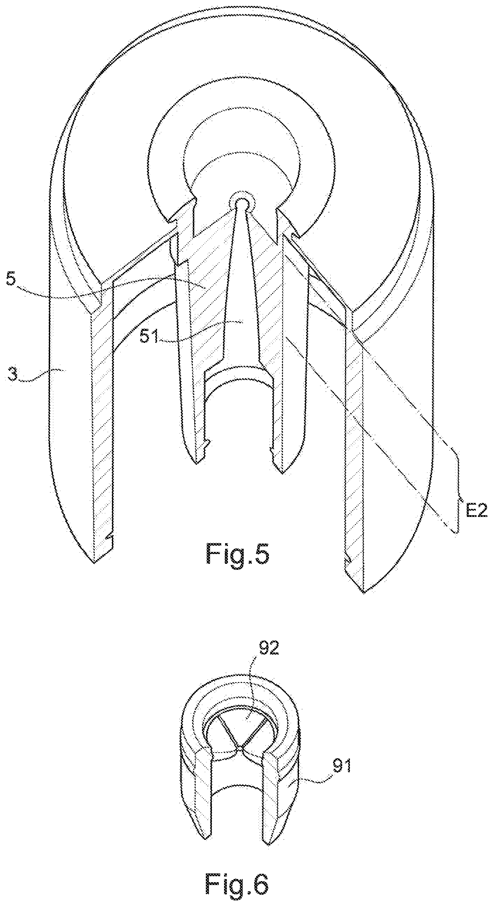

La figure 5 représente, selon une vue en trois dimensions, le corps tournant 3 et le cylindre percé 5 qui y est fixé. La vue est tronquée de sorte à montrer l'intérieur de ces pièces.

La restriction de section formant le deuxième étage E2 du dispositif de rupture d'éléments solides est progressive, et tend à comprimer les éléments solides jusqu'à éclatement. Ainsi, la section la plus restreinte du deuxième étage, qui correspond ici à la section de sortie du conduit 51, est telle qu'elle ne permet pas le passage sans une déformation importante, entrainant un éclatement des éléments solides B. Par exemple, la dimension maximale de la section la plus restreinte du deuxième étage (c'est à dire son diamètre si la restriction est circulaire, grand diamètre si elle est, selon une forme non-revendiquée, elliptique, etc.) est inférieure au diamètre des éléments solides si ceux-ci sont sensiblement sphériques. Par exemple, la section la plus restreinte (ici la section de sortie du deuxième étage E2) peut être circulaire et avoir un diamètre de 0,6mm. Le premier étage peut présenter une section telle qu'elle peut permettre le passage de billes jusqu'à 0,8mm de diamètre.

Les billes ont par exemple un diamètre de l'ordre de 1mm, avec potentiellement une certaine dispersion dimensionnelle.

La conjonction des effets du premier étage E1 et du deuxième étage E2 permet un éclatement d'un grand nombre d'éléments solides. Par exemple, si un élément solide a sa paroi affaiblie mais pas rompue par le premier étage E1, celle-ci est facilement éclatée par compression dans le deuxième étage du fait de son affaiblissement préalable. En outre, les éléments solides présents dans le produit peuvent présenter une certaine dispersion de leurs dimensions. Le premier étage E1 peut permettre en particulier la rupture des éléments solides les plus gros, tandis que le deuxième étage E2 permet la rupture des éléments solides les plus petits, qui n'ont pas été éclatés par le premier étage E1. Du fait de la rupture des éléments les plus gros dans le premier étage E1, la perte de charge qui serait liée à l'obstruction de la restriction de section du deuxième étage E2 par des gros éléments solides est limitée.

Dans la variante de l'invention ici représentée, un clapet anti-retour 91 est disposé entre la sortie du conduit 51 et l'embout d'application 4. Le clapet anti-retour 91 permet de protéger le produit P contenu dans le dispositif de l'atmosphère extérieure. Le clapet anti-retour 91 permet également, dans une certaine mesure, d'éviter que le corps tournant ne soit actionné dans le sens de rotation tendant à ré-aspirer du produit vers le fût intérieur.

Le clapet anti-retour 91 peut également aider à l'éclatement des éléments solides présents dans le produit P avant sa distribution. En effet, le clapet anti-retour 91 peut présenter une faible section de sortie, et, selon certains modes de réalisation tel que celui représenté à la figure 6, des membranes ou ailettes 92 tendant à se refermer et à rompre les parois des éléments solides ayant le cas échéant traversé les premier et deuxième étages du dispositif de rupture d'éléments solides sans que leur paroi ne soit rompue.

De manière avantageuse le clapet anti-retour contribue à l'étanchéité générale du dispositif de distribution lorsque ce dernier est en position inversée (embout d'application 4 orienté vers le bas), par exemple dans le sac à main d'une utilisatrice.

L'invention est particulièrement adaptée à la distribution d'un produit P cosmétique ou de soin dans lequel il convient de libérer un fluide juste avant la distribution dudit produit, par exemple un brillant à lèvres, dans lequel on libère un ingrédient pour améliorer le brillant obtenu.

Le mécanisme à piston fixe et corps rotatif du dispositif décrit précédemment est particulièrement bien adapté à un dispositif conforme à l'invention, car il permet un contrôle aisé de la quantité de produit délivrée. Comparativement à un système classique à pompe, le risque de blocage de la pompe par le produit est éliminé.

Sur ce principe, d'autres mécanismes peuvent être mis en œuvre, par exemple dans lesquels le corps tournant comporte une partie filetée sur sa surface intérieure, tandis que le réservoir est lié à ou comporte une bague présentant un filetage mâle correspondant.

L'invention peut mettre en œuvre, de manière générale, tout type de mécanisme permettant l'expulsion du produit P via un conduit de guidage dans lequel sont ménagés les deux étages d'un dispositif de rupture d'éléments solides.

Il est notable que l'embout de distribution 4 peut présenter des formes diverses, selon le type de produit et la zone d'application visée.

L'embout de distribution 4 peut présenter une surface d'application sensiblement conique, ou sensiblement plane et inclinée vis-à-vis de l'axe principal (A) du dispositif, comme dans l'exemple de la figure 1, ou encore être bombé.

Les dispositifs présentés sont notamment adaptés à l'application d'un brillant à lèvres, plus couramment désigné par le terme anglophone « gloss ».

L'invention ainsi développée offre un dispositif permettant la distribution d'un produit cosmétique ou de soin stocké sous forme hétérogène, c'est-à-dire comportant des éléments solides. Du fait de la présence d'au moins deux étages d'un dispositif de rupture des éléments solides, disposés en ligne et ne présentant pas d'élément du type filtre susceptible d'être colmaté, l'invention permet tout à la fois la rupture d'une grande partie (sinon de la totalité) des éléments solides présents dans le produit distribué, tout en limitant le risque de colmatage comparativement aux dispositifs connus dans l'état de la technique.

## Revendications

1. Dispositif de distribution d'un produit (P) cosmétique ou de soin liquide, semi-fluide ou pâteux, stocké sous forme hétérogène comportant des éléments solides (B) en suspension pouvant être rompus mécaniquement pour libérer un fluide qu'ils contiennent, comportant :
• un réservoir (1) destiné au stockage du produit ;
• un embout de distribution (4) du produit ;
• un conduit de guidage permettant le transport du produit entre le réservoir (1) et l'embout de distribution (4); et
• des moyens adaptés à provoquer la circulation du produit du réservoir (1) vers l'embout, via le conduit de guidage, le conduit de guidage comportant un dispositif de rupture des éléments solides (B) ;
**caractérisé en ce que** le dispositif de rupture d'élément solides comporte :
un premier étage (E1), en sortie du réservoir (1), formant une première portion du conduit de guidage et comportant une section transversale qui évolue progressivement et qui présente en sortie des arêtes saillantes à l'intérieur dudit conduit de guidage ; et
un deuxième étage (E2), à proximité de l'embout de distribution, formant une deuxième portion du conduit de guidage et présentant une restriction de section progressive, ladite deuxième portion du conduit de guidage, formée dans le deuxième étage (E2) du dispositif de rupture, est en forme de tronc de cône.

2. Dispositif selon la revendication 1, comportant en outre, en aval du deuxième étage (E2) du dispositif de rupture, un clapet anti-retour (91).

3. Dispositif selon la revendication 2, dans lequel le clapet anti-retour (91) présente une embouchure de sortie du produit de section inférieure à la section de sortie du premier étage (E1) et à la section la plus restreinte du deuxième étage (E2).

4. Dispositif selon l'une des revendications 1 à 3, dans lequel la section de sortie du premier étage (E1) est en étoile.

5. Dispositif selon l'une des revendications précédentes, dans lequel la restriction de section du deuxième étage (E2) correspond à une restriction comprise entre 50% et 95% de la surface de la section d'entrée du deuxième étage (E2).

6. Dispositif selon l'une des revendications précédentes,
ledit dispositif comportant un corps tournant (3) allongé et creux et un fût extérieur (2) allongé et creux aboutés l'un à l'autre, et mobile en rotation l'un par rapport à l'autre,
l'embout de distribution (4) étant fixé en bout du corps tournant (3),
lesdits corps tournant (3) et fût extérieur (2) formant un volume intérieur dans lequel sont ménagés :
un cylindre percé (5), comportant le conduit de guidage; et
un fût intérieur, comportant une extrémité ouverte par laquelle est introduit le cylindre percé (5) et une extrémité fermée de sorte à former le réservoir (1),
lequel dispositif comporte un mécanisme de transformation d'une rotation du corps tournant (3) par rapport au fût extérieur (2) en un mouvement de translation du fût intérieur vis-à-vis du cylindre percé (5) dans le volume intérieur formé par lesdits corps tournant (3) et fût extérieur (2).

7. Dispositif selon la revendication 6, comportant en outre un piston (52) monté fixement à une extrémité du cylindre percé (5) introduite par l'extrémité ouverte du fût intérieur et comportant une partie allongée formant la première portion du conduit de guidage dans le cylindre percé (5), dans lequel le piston (52) comporte une ouverture centrale (53) formant le premier étage (E1) du dispositif de rupture.

8. Ensemble comportant un dispositif selon l'une des revendications précédentes, et un produit cosmétique ou de soin comportant des éléments solides (B) en suspension, pouvant être rompus mécaniquement pour libérer un fluide qu'ils contiennent, ledit produit cosmétique ou de soin étant stocké dans le réservoir (1) du dispositif.

9. Ensemble selon la revendication 8, dans lequel les éléments solides (B) sont des billes sensiblement sphériques de diamètre inférieur à la dimension maximale de la section la plus restreinte du deuxième étage (E2).

## Patentansprüche

1. Vorrichtung zur Ausgabe eines flüssigen, halbflüssigen oder pastenartigen kosmetischen oder Pflegeprodukts (P), das in heterogener Form gespeichert ist und in Suspension befindliche Feststoffelemente (B) umfasst, die mechanisch zerbrochen werden können, um ein Fluid, das sie enthalten, freizugeben, umfassend:
• einen Behälter (1), der zur Speicherung des Produkts bestimmt ist;
• ein Ausgabeendstück (4) für das Produkt;
• einen Führungskanal, der den Transport des Produkts zwischen dem Behälter (1) und dem Ausgabeendstück (4) ermöglicht; und
• Mittel, die dazu eingerichtet sind, das Strömen des Produkts vom Behälter (1) über den Führungskanal zum Endstück zu bewirken, wobei der Führungskanal eine Vorrichtung zum Zerbrechen der Feststoffelemente (B) umfasst;
**dadurch gekennzeichnet, dass** die Vorrichtung zum Zerbrechen der Feststoffelemente umfasst:
eine erste Stufe (E1) am Ausgang des Behälters (1), die einen ersten Abschnitt des Führungskanals bildet und einen Querschnitt aufweist, der sich allmählich vergrößert und der am Ausgang Kanten aufweist, die ins Innere des Führungskanals hineinragen; und
eine zweite Stufe (E2) in der Nähe des Ausgabeendstücks, die einen zweiten Abschnitt des Führungskanals bildet und eine allmähliche Querschnittsverminderung aufweist, wobei der zweite Abschnitt des Führungskanals, der in der zweiten Stufe (E2) der Brechvorrichtung ausgebildet ist, eine Kegelstumpfform hat.

2. Vorrichtung nach Anspruch 1, welche außerdem, stromabwärts der zweiten Stufe (E2) der Brechvorrichtung, ein Rückschlagventil (91) umfasst.

3. Vorrichtung nach Anspruch 2, wobei das Rückschlagventil (91) eine Austrittsmündung für das Produkt mit einem Querschnitt aufweist, der kleiner als der Ausgangsquerschnitt der ersten Stufe (E1) und als der kleinste Querschnitt der zweiten Stufe (E2) ist.

4. Vorrichtung nach einem der Ansprüche 1 bis 3, wobei der Ausgangsquerschnitt der ersten Stufe (E1) sternförmig ist.

5. Vorrichtung nach einem der vorhergehenden Ansprüche, wobei die Querschnittsverminderung der zweiten Stufe (E2) einer Verkleinerung entspricht, die zwischen 50 % und 95 % der Fläche des Eintrittsquerschnitts der zweiten Stufe (E2) beträgt.

6. Vorrichtung nach einem der vorhergehenden Ansprüche,
wobei die Vorrichtung einen lang gestreckten und hohlen rotierenden Körper (3) und einen lang gestreckten und hohlen äußeren Schaft (2), die aneinanderstoßen und relativ zueinander drehbeweglich sind, umfasst,
wobei das Ausgabeendstück (4) am Ende des rotierenden Körpers (3) befestigt ist,
wobei der rotierende Körper (3) und der äußere Schaft (2) ein Innenvolumen bilden, in dem angeordnet sind:
ein durchbohrter Zylinder (5), der den Führungskanal umfasst; und
ein innerer Schaft, der ein offenes Ende aufweist, über das der durchbohrte Zylinder (5) eingeführt wird, und ein geschlossenes Ende, um den Behälter (1) zu bilden,
wobei die Vorrichtung einen Mechanismus zur Umwandlung einer Rotation des rotierenden Körpers (3) bezüglich des äußeren Schafts (2) in eine Translationsbewegung des inneren Schafts gegenüber dem durchbohrten Zylinder (5) in dem Innenvolumen, das von dem rotierenden Körper (3) und dem äußeren Schaft (2) gebildet wird, umfasst.

7. Vorrichtung nach Anspruch 6, welche außerdem einen Kolben (52) umfasst, der fest an einem Ende des durchbohrten Zylinders (5) angebracht ist, das über das offene Ende des inneren Schafts eingeführt wird, und der einen lang gestreckten Teil umfasst, der den ersten Abschnitt des Führungskanals in dem durchbohrten Zylinder (5) bildet, wobei der Kolben (52) eine zentrale Öffnung (53) aufweist, welche die erste Stufe (E1) der Brechvorrichtung bildet.

8. Anordnung, welche eine Vorrichtung nach einem der vorhergehenden Ansprüche umfasst, sowie ein kosmetisches oder Pflegeprodukt, das in Suspension befindliche Feststoffelemente (B) umfasst, die mechanisch zerbrochen werden können, um ein Fluid, das sie enthalten, freizugeben, wobei das kosmetische oder Pflegeprodukt in dem Behälter (1) der Vorrichtung gespeichert ist.

9. Anordnung nach Anspruch 8, wobei die Feststoffelemente (B) im Wesentlichen kugelförmige Elemente mit einem Durchmesser sind, der kleiner als die maximale Abmessung des kleinsten Querschnitts der zweiten Stufe (E2) ist.

## Claims

1. Dispensing device for a liquid, semi-fluid or paste-like cosmetic or care product (P) stored in heterogeneous form including solid elements (B) in suspension which can be mechanically ruptured to release a fluid contained therein, including:
• a reservoir (1) for storing the product;
• a dispensing nozzle (4) for the product;
• a guide duct for conveying the product between the reservoir (1) and the dispensing nozzle (4); and
• means designed to cause the product to flow from the reservoir (1) to the nozzle, via the guide duct, the guide duct including a device for rupturing the solid elements (B);
**characterized in that** the device for rupturing solid elements includes:
a first stage (E1), at the outlet of the reservoir (1), forming a first portion of the guide duct and having a cross-section which changes gradually and which has edges at the outlet that project into said guide duct; and
a second stage (E2), close to the dispensing nozzle, forming a second portion of the guide duct and having a gradually narrowing cross-section, said second portion of the guide duct, formed in the second stage (E2) of the rupturing device, is in the shape of a truncated cone.

2. Device according to Claim 1, further including a non-return valve (91) downstream of the second stage (E2) of the rupturing device.

3. Device according to Claim 2, wherein the non-return valve (91) has an outlet opening for the product having a smaller cross-section than the outlet cross-section of the first stage (E1) and the narrowest cross-section of the second stage (E2).

4. Device according to one of Claims 1 to 3, wherein the outlet cross-section of the first stage (E1) is star-shaped.

5. Device according to one of the preceding claims, wherein the narrowing of the cross-section of the second stage (E2) is a narrowing of between 50% and 95% of the surface area of the inlet cross-section of the second stage (E2).

6. Device according to one of the preceding claims,
said device including an elongated hollow rotating body (3) and an elongated hollow outer shaft (2) abutting each other and movable in rotation relative to each other,
the dispensing nozzle (4) being fixed at the end of the rotating body (3),
said rotating body (3) and outer shaft (2) forming an internal volume in which are arranged:
a bored cylinder (5), including the guide duct; and
an inner shaft, having an open end through which the bored cylinder (5) is inserted and a closed end to form the reservoir (1),
this device includes a mechanism for transforming a rotation of the rotating body (3) with respect to the outer shaft (2) into a translational movement of the inner shaft with respect to the bored cylinder (5) in the internal volume formed by said rotating body (3) and said outer shaft (2).

7. Device according to Claim 6, further including a piston (52) fixedly mounted at one end of the bored cylinder (5) inserted through the open end of the inner shaft and having an elongate portion forming the first portion of the guide duct in the bored cylinder (5), in which the piston (52) has a central opening (53) forming the first stage (E1) of the rupturing device.

8. Assembly including a device according to one of the preceding claims, and a cosmetic or care product including solid elements (B) in suspension, which can be mechanically ruptured to release a fluid contained therein, said cosmetic or care product being stored in the reservoir (1) of the device.

9. Assembly according to Claim 8, wherein the solid elements (B) are substantially spherical balls of diameter less than the maximum dimension of the narrowest cross-section of the second stage (E2).
